# EUROPEAN PATENT APPLICATION

(11) **EP 2 441 407 A1**
(43) Date of publication of application: **18.04.2012**
(21) Application number: 09845851.6
(22) Date of filing: 09.06.2009
(51) Int. Cl.: A61B 18/14, A61B 17/32, A61B 17/34, A61B 17/70, A61L 29/04

(54) **DIRECTION-CONTROLLABLE ELECTRODE BODY FOR SELECTIVELY REMOVING BODILY TISSUE, AND GUIDE PIPE**

(60) Divisional application: 12153725.2
(71) Applicant: U & I Corporation, Uijeongbu-si Gyeonggi-do 480-050 (KR); Korea University Industrial&Academic Collaboration Foundation, Seoul 136-713 (KR)
(72) Inventor: LEE, Sang-Heon, Seoul 135-270 (KR); CHO, Sung-Youn, Uijeongbu-si Gyeonggi-do 480-901 (KR); KANG, Bong-Su, Dongducheon-si Gyeonggi-do 483-755 (KR); KIM, Sang-Woon, Uijeongbu-si Gyeonggi-do 480-847 (KR); KOO, Ja-Kyo, Seoul 139-220 (KR); KIM, Jong-Tack, Jeonju-si Jeollabu-do 561-803 (KR)
(74) Representative: Collin, Jérôme
(86) International application number: PCT/KR2009/003090
(87) International publication number: WO 2010/143757

(57) **Abstract**

Disclosed is a direction controllable electrode body, comprising a flexible body which comprises: a first electrode, comprising a body, a first cap joined to one end of the body, and a first electrode line connected to the other end of the body; an insulator in partial contact with the body and the first cap of the first electrode, said insulator functioning to insulate the first electrode from the second electrode; a second electrode, comprising a first ring in contact with the insulator, and a second electrode line connected to one end of the first ring; and a direction controller, comprising a first direction controlling wire communicating with the first electrode or the second electrode to control direction of the electrode body. Also, a guide pipe is provided for leading the electrode body to a target tissue.

## Description

### Technical Field

The present invention relates to an electrode body useful for locally removing bodily tissue using radiofrequency (RF). More particularly, the present invention relates to a direction controllable electrode body which can readily penetrate into a rigid fibroid material in the body and advance to a target tissue in a controlled manner within the material using a controlling wire. Also, the present invention is concerned with a guide pipe which can lead the electrode body to a target tissue of the body.

### Background Art

Spinal disc herniation is a medical condition affecting the spine, in which an intervertebral disc protrudes to put pressure on the nerve located near the disc, resulting in low back and radicular pain. The intervertebral discs consist of an outer annulus fibrosus, which surrounds the inner nucleus pulposus. After the annulus fibrosus is torn by physical impact, when the disc is under a lot of pressure, generated by the body weight upon standing or by some excessive impact, the nucleus pulposus is allowed to bulge out through the tear in the annulus fibrosus. In addition, a high internal pressure on the intervertebral disc is transferred to the outer layer of the disc, so that the disc protrudes. This medical condition is called spinal disc herniation. If the protruded disc does not come back, but is maintained, it puts pressure on the nerve to cause lumbar pain.

Spinal disc herniation may be cured by surgery. As many as 30% of the surgical cases performed for spinal disc herniation, however, are reported to yield no therapeutic effects. Further, because back surgery is apt to be accompanied by the ablation of a spinal nerve, 5 - 10% of the patients who undergo back surgery suffer from failed back surgery syndrome (F.B. S. S) for the rest of their lives.

Another way to treat intervertebral disc herniation is a restoration method by which the nucleus pulposus in an intervertebral disc herniation is removed to reduce the internal pressure so that the protruded disc spontaneously recedes. This method is a non-surgical treatment method characterized by the use of radiofrequency energy to ablate the disc hernia. When radiofrequency is applied to a radiofrequency electrode which is routed to the internal tissue of an intervertebral disc, an electric field of high energy is formed around the electrode and dissociates the constituents of the nucleus pulposus into negatively and positively charged ions to remove the herniated disc. Compared to a surgical method, the method of using a radiofrequency electrode to remove bodily tissue has the advantages of reducing the hospitalization period, decreasing medical expenses, and avoiding the aftereffects of surgery.

Usually, the radio frequency used in this medical treatment has an oscillation rate of from 100 to 20,000 kHz. The use of a radiofrequency electrode to ablate bodily tissues or remove intravascular occlusions is disclosed in U. S. Patent No. 6,554,827 B2.

While lower back pain is caused by a part of the intervertebral disc being herniated and pressing adjacent nerve roots, hydrostatic pressure is put on the outer annulus fibrosus by the inner nucleus pulposus. Because the fluidity of the inner nucleus pulposus in the hernia is not large, the intervertebral disc is kept in the herniated state, continuing to compress the nerve roots and cause lower back pain. For non-surgical treatment, first, radiofrequency electrode tips are inserted into the outer tissue of the disc hernia with the aid of a guide pipe. Application of a radiofrequency generates an electric field between the two electrodes, dissociating the internal composition into negatively and positively charged particles which form an electrically neutral medium. Thus, the internal pressure of the intervertebral disc is reduced to make the bulge-out of the disc to recede. In addition to the indirect restoration by pressure reduction, the electrode tip of the guide pipe which is located in the hernia of the intervertebral disc can directly ablate the hernia tissue to remove the nerve compression, thus curing the lower back and radicular pain.

Because they are straight, most conventional radiofrequency electrodes are disadvantageous in that their accessible regions are limited because they can only reach the sites which are in a straight line with the insertion position of the electrode. Particularly when a spinal disc herniation is treated, the position at which the radiofrequency electrode tip is inserted into the body is very restricted because of the spine and spinal nerves. Accordingly, it is very difficult to ablate the tissue opposite to the insertion position of the radiofrequency electrode. The method of using a radiofrequency electrode is based on the principle in which tissue is removed to generate a negative pressure which acts in turn to restore a herniated intervertebral disc. Thus, unless removing the tissue around the hernia, the therapeutic efficiency of the method is very poor.

Meanwhile, there are radiofrequency electrodes that have a bent end portion that is bent at a constant curvature before being inserted into the body. The restoring force of the bent electrodes causes them to reach a site which is difficult to access in a straight manner after proceeding through a guide pipe. However, once entering the body, it is difficult to restore the bent portion. In fact, these bent electrodes are not widely used because their success depends on experience and guesswork. In addition, the bent electrodes, although sufficiently restorative, are difficult to accurately position on the herniated intervertebral disc which differs from one individual to another.

Also disclosed is an electrode that has an end portion which is made of a flexible polymer material and can be readily bent. However, it is also difficult to accurately position this electrode on the herniated spinal disc. Further, the polymer material may melt upon the application of radiofrequency.

In patients who have suffered from a herniated spinal disc for a long period of time, a neck is generated between the annulus fibrosus and the protruded portion. As time passes, tissue may grow into the neck, forming an occluded firm fibroid. In this case, the nucleus pulposus in the herniated spinal disc is difficult to remove because the electrode tip cannot penetrate into the occluded firm fibroid.

### Disclosure

### Technical Problem

The present invention aims to provide a medical device which has a direction controlling function so as to readily approach a target tissue within the body and which can selectively remove the target to achieve a therapeutic effect.

### Technical Solution

In accordance with an aspect thereof, the present invention provides a direction controllable electrode body, comprising a flexible body which comprises: a first electrode, comprising a body, a first cap joined to one end of the body, and a first electrode line connected to the other end of the body; an insulator in partial contact with the body and the first cap of the first electrode, said insulator functioning to insulate the first electrode from the second electrode; a second electrode, comprising a first ring in contact with the insulator, and a second electrode line connected to one end of the first ring; and a direction controller, comprising a first direction controlling wire communicating with the first electrode or the second electrode to control direction of the electrode body.

In accordance with another aspect thereof, the present invention provides a method for treating disc disease, comprising : a step of (A) approaching a location of a lesion causing back pain by controlling the direction controllable electrode body of claim 1; and at least one step selected from the group consisting of (B) searching a nerve responsible for the pain by stimulating the lesion with the electrode body; (C) treating the disc disease by coagulating the lesion with the electrode body; and (D) treating the disc disease by ablating a disc tissue causing the pain with the electrode body

In accordance with a further aspect thereof, the present invention provides a guide pipe, comprising: a second flexible protecting pipe; a second cap conjugated to one end of the second flexible protecting pipe; a second direction controlling wire, extending from the second cap to the other end of the second flexible protecting pipe; and a hollow, second direction operator communicating with the second direction controlling wire.

### Advantageous Effects

After being inserted into the body, the electrode body of the present invention can be controllably positioned by bending the flexible body with the direction controlling wire. Being capable of approaching a target tissue within the body in a controlled manner, the electrode body can selectively get rid of the target tissue. Further, the electrode body can readily invade a rigid fibroid material so that it can be applied to the treatment of spinal disc herniation which has been fixed for a long period of time.

### Description of Drawings

FIG 1. is a perspective view showing a direction controllable electrode body according to an embodiment of the present invention.

FIG. 2 is a perspective view showing a flexible body of the direction controllable electrode.

FIG. 3 is a perspective view showing constituents used in the flexible body of the direction controllable electrode body.

FIG. 4 is a schematic view showing a process of controlling the direction and position of the electrode body inserted into an intervertebral disc.

FIG. 5 is a perspective view the electrode body according to an embodiment of the present invention which is associated with a direction controller.

FIG. 6 is a perspective view showing a part of an electrode puncture into which the electrode body according to an embodiment of the present invention will be inserted.

FIG. 7 is a side view showing a curved needle having a conical cap at one end.

FIG. 8 is a side view showing a part of the curved needle of FIG. 7.

FIG. 9 is a cross-sectional view showing a part of the curved needle of FIG. 7.

FIG. 10 is an expanded side view of the conical cap of the curved needle.

FIG. 11 is a side view of a curved needle that has a groove in one side and a wire installed within the groove, the wire being manipulated to control the direction of the curved needle.

FIG. 12 is a side view of a curved needle having a groove formed in one side and a cylindrical flexible region adjacent to the groove.

FIG. 13 is a side view of a curved needle having a groove in one side and a mesh-type flexible region adjacent to the groove.

FIG. 14 is a side view of a curved needle having a groove in one side and a coil-type flexible region adjacent to the groove.

FIG. 15 is a side view of a curved needle having a groove in one side and an articular flexible region adjacent to the groove.

FIG. 16 is a schematic view showing a curved needle having a groove in one side and a flexible region adjacent to the groove which is inserted into the disc between the fifth lumbar vertebra L5 and the sacrum S 1.

FIG. 17 is of side views showing protectors for protecting a body tissue from the heat generated by the electrode body.

FIG. 18 is a graph showing a waveform generated when one alternating current is used.

FIG. 19 is a graph showing waveforms generated when two alternating currents are used with a phase difference.

FIG. 20 is a perspective view of a guide pipe according to an embodiment of the present invention.

FIG. 21 is an enlarged cross-sectional view showing the structure of the guide pipe of FIG. 20.

FIG. 22 is a perspective view showing a guide pipe into which a trocar is inserted.

FIG. 23 is a cross-sectional view showing a guide pipe comprising a second direction controlling wire extended from a third ring, with a trocar inserted thereinto, in accordance with an embodiment of the present invention.

FIG. 24 is a cross-sectional view of a guide pipe in which a groove is formed between the second cap and the third ring, but not at one end of the second cap, in one side.

FIG. 25 is of photographs showing a process of assembling the guide pipes of the present invention into a larger guide pipe.

### Best Mode

The present invention provides a direction controllable electrode body, comprising a flexible body which comprises: a first electrode, comprising a body, a first cap joined to one end of the body, and a first electrode line connected to the other end of the body; an insulator in partial contact with the body and the first cap of the first electrode, said insulator functioning to insulate the first electrode from the second electrode; a second electrode, comprising a first ring in contact with the insulator, and a second electrode line connected to one end of the first ring; and a direction controller, comprising a first direction controlling wire communicating with the first electrode or the second electrode to control direction of the electrode body

The direction controllable electrode body may further comprise a rigid body joined to the flexible body.

In this case, the direction controllable electrode body may further comprise a first direction operator combined with the rigid body.

In the direction controllable electrode body, the body of the first electrode is integrated with the first cap.

In an embodiment of the direction controllable electrode body, the first electrode and the second electrode are independently made of a material selected from the group consisting of stainless steel, general alloy steel, titanium steel and shape memory steel.

The insulator may be made of a material selected from the group consisting of ceramic, silicon, a fluororesin, a heat-shrinkable polymer, and combinations thereof.

The ceramic is Al2O3, the silicon is Si02, and the heat-shrinkable polymer is selected from the group consisting of polytetrafluoroethylene (PTFE), tetrafluoroethylene-hexafluoropropylene (FEP), tetrafluoroethylene-perfluoroalkylvinylether (PFA), ethylene-tetrafluoroethylene (ETFE), polyester (PET), polyether ether ketone (PEEK), and combinations thereof.

In the direction controllable electrode body, the first direction controlling wire has an electric resistance of from 0.1 µΩ to 5 Ω and a tensile strength of from 100 MPa to 20 GPa and is used both to control direction of the electrode body and to serve as a power supply line, whereby the electrode can be reduced in diameter.

The first direction controlling wire may be made of a material selected from the group consisting of stainless steel, titanium, cobalt-chrome alloy, platinum, silver and combinations thereof.

In addition, the first direction controlling wire may be coated with enamel.

In the direction controllable electrode body, the direction controller may comprise a second ring connected to the first electrode or the second electrode.

This second ring is connected to the first cap of the first electrode or the first ring of the second electrode.

The flexible body may further comprise a first flexible protecting pipe for protecting the first electrode, the insulator, the second electrode and the direction controller.

In an embodiment of the direction controllable electrode body, the first flexible protecting pipe has a coil or articular structure.

The first flexible protecting pipe may be made of a soft polymer.

This soft polymer may have a shore hardness of from 40 to 75 shore D.

In this regard, the soft polymer may be selected from the group Pebax 4533, Pebax 5533, Pebax 7233 (Atochem), Nylon-12, ultra high-molecular weight polyethylene (UHMP), polytetrafluoroethylene (PTFE), tetrafluoroethylene-hexafluoropropylene (FEP), polyesteramide (PEA), ethylene-tetrafluoroethylene (ETFE), polyvinylidenefluoride (PVDF), polyether ether ketone (PEEK), low-density polyethylene (LDPE), high-density polyethylene (HDPE) and combinations thereof.

Moreover, the direction controllable electrode body may be inserted into a groove formed in one side of a curved needle, whereby the electrode body can be directionally controlled as the curved needle is operated.

The curved needle may a flexible region adjacent to the groove.

The flexible region may be made of a material selected from the group consisting of a polymer, a metal and a composite of a polymer and a metal.

In this context, the polymer may be selected from the group consisting of ultra high-molecular weight polyethylene (UHMP), polytetrafluoroethylene (PTFE), tetrafluoroethylene-hexafluoropropylene (FEP), polyesteramide (PEA), ethylene-tetrafluoroethylene (ETFE), polyvinylidenefluoride (PVDF), polyether ether ketone (PEEK), low-density polyethylene (LDPE), high-density polyethylene (HDPE) and combinations thereof, and the metal is stainless steel.

The structure of the flexible region is selected from the group consisting of a cylindrical type, a meshed cylindrical type, a coil type and an articular type.

In this electrode body, one side of the cylindrical type structure of the flexible region is made of a metal while the other side is made of a polymer, so that the curved needle is bent in the direction of the polymer side.

A flexible region with the articular type of structure is composed of a plurality of triangular rings.

In this structure, each of the triangular rings has an insertion hole or a recess formed at an upper and a lower position thereof into which a direction controlling wire is inserted.

The direction controllable electrode body may be associated with a protector for protecting adjacent body tissue from heat generated by the electrode body, and the protector is composed of a protecting membrane and a support for supporting the protecting membrane.

The protecting membrane may have a mesh type structure or a cruciform type structure.

The protecting membrane may be made of a material selected from the group consisting of polytetrafluoroethylene (PTFE), polyethylene (PE), polyether ether ketone (PEEK), tetrafluoroethylene-hexafluoropropylene (FEP), tetrafluoroethylene-perfluoroalkylvinylether (PFA), ethylene-tetrafluoroethylene (ETFE), polyimide (PI), polyester (PET), polyamide (PA) and combinations thereof

In the direction controllable electrode body, the support may be a Y-shaped tube.

Meanwhile, the support may be made of a material selected from the group consisting of a polymer, a metal and a composite thereof.

The membrane may be made of a polymer selected from the group consisting of polytetrafluoroethylene (PTFE), polyethylene (PE), polyether ether ketone (PEEK), tetrafluoroethylene-hexafluoropropylene (FEP), tetrafluoroethylene-perfluoroalkylvinylether (PFA), ethylene-tetrafluoroethylene (ETFE), polyimide (PI), polyester (PET), polyamide (PA), and combinations thereof.

For use in the support, the metal may be stainless steel.

Also, the present invention provides a method for treating disc disease, comprising : a step of (A) approaching a location of a lesion causing back pain by controlling the direction controllable electrode body of claim 1; and at least one step selected from the group consisting of (B) searching a nerve responsible for the pain by stimulating the lesion with the electrode body; (C) treating the disc disease by coagulating the lesion with the electrode body; and (D) treating the disc disease by ablating a disc tissue causing the pain with the electrode body.

The step (A) may be followed by step (D).

In an alternative embodiment, the steps may be conducted in the order of (A), (B) and (C).

In another alternative, the step (A) may be conducted, followed by sequentially conducting steps (D), (B) and (C).

In the method, the step (A) of advancing the direction controllable electrode body to a lesion comprises: inserting the electrode body into the annulus fibrosus of an intervertebral disc of interest, with the electrode body staying spread; and controllably bending the flexible body of the electrode body in a desired direction to position the cap and the ring on the lesion.

In the method, the step (B) of stimulating the lesion to search for a nerve responsible for the pain comprises applying an alternating current of 1 Hz to 300 Hz at a voltage of from 0.1 to 3.0 V to the electrode body to detect a nerve responsible for the pain.

The step (C) of coagulating the lesion may be conducted by electrocautery in which an alternating current of 300 - 500 kHz is applied to the electrode body to remove the nerve.

Further, the present invention provides a guide pipe, comprising: a second flexible protecting pipe; a second cap conjugated to one end of the second flexible protecting pipe; a second direction controlling wire, extending from the second cap to the other end of the second flexible protecting pipe; and a hollow, second direction operator communicating with the second direction controlling wire.

In this guide pipe, the second flexible protecting pipe may have a coil type or articular type of structure.

The second flexible protecting pipe may be made of a soft polymer.

This soft polymer may have a shore hardness of 40 ∼ 75 shore D.

In an embodiment of the guide pipe, the soft polymer may be selected from the group consisting ofPebax 4533, Pebax 5533, Pebax 7233 (Atochem), Nylon-12, ultra high-molecular weight polyethylene (UHMP), polytetrafluoroethylene (PTFE), tetrafluoroethylene-hexafluoropropylene (FEP), polyesteramide (PEA), ethylene-tetrafluoroethylene (ETFE), polyvinylidenefluoride (PVDF), polyether ether ketone (PEEK), low-density polyethylene (LDPE), high-density polyethylene (HDPE), and combinations thereof.

The second direction controlling wire may have an electric resistance of from 0.1 µΩ to 5 Ω and a tensile strength of from 100 MPa to 20 GPa and is used both to control direction of the electrode body and to serve as a power supply line, whereby the diameter of the electrode can be reduced.

Also, the second direction controlling wire may be made of a material selected from the group consisting of stainless steel, titanium, cobalt-chrome alloy, platinum, silver and combinations thereof.

The second direction controlling wire may be coated with enamel.

The guide pipe may contain a trocar inside it.

In an embodiment, the guide pipe may contain the direction controllable electrode body of the present invention therein, so that the electrode body can be allowed to make a turnaround in a controlled manner established by the guide pipe.

The above guide pipe has a radius of curvature and a length and serves as one of the many segments which are assembled into a larger guide pipe.

The guide pipe may have a radius of curvature of from 10 to 5000 mm and a length of from 0.5 to 3.5 mm.

In an embodiment, the guide pipe may contain the direction controllable electrode body of the present invention therein.

### Mode for Invention

Reference should now be made to the drawings, in which the same reference numerals are used throughout the different drawings to designate the same or similar components.

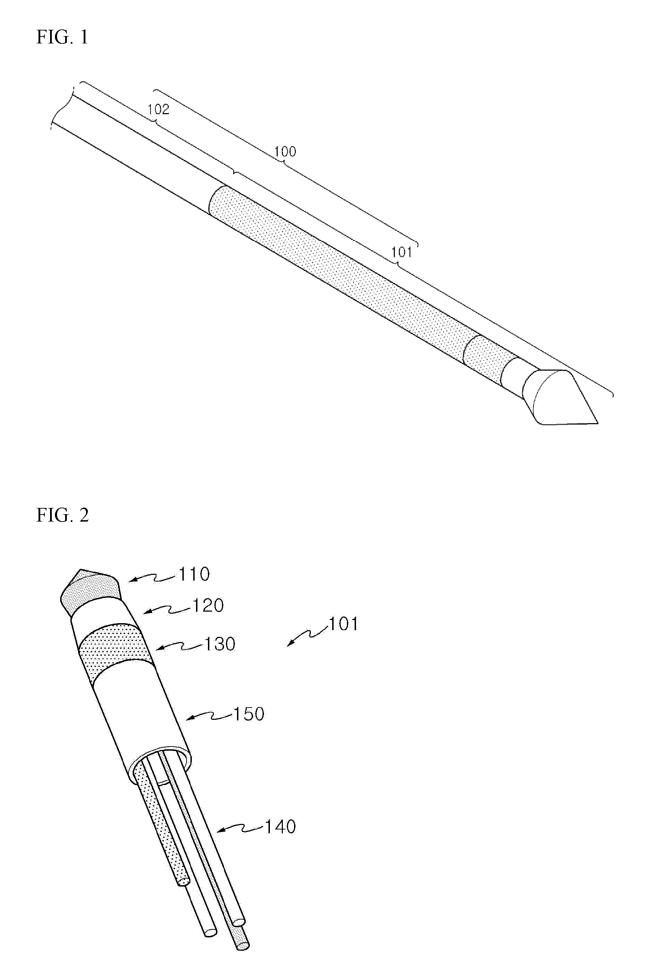

FIG. 1 is a perspective view showing a direction-controllable electrode body in accordance with an embodiment of the present invention.

With reference to FIG. 1, the direction-controllable electrode body 100 in accordance with an embodiment of the present invention comprises a flexible body 101.

When the electrode body is applied to the body, the flexible body 101 is in direct contact with the body and can be directionally controlled within the body.

To be easily inserted into the body, the electrode 100 may further comprise a rigid body connected to one end of the flexible body 101. The rigid body 102 may function to support and protect the flexible body 101. The rigid body 102 may be made of a polymer which has a high hardness or which is inserted into a stainless steel pipe conferring bending resistance to the polymer. Examples of the polymer with high hardness include PEBAX, ultra high-molecular weight polyethylene (UHMP), polytetrafluoroethylene (PTFE), tetrafluoroethylene-hexafluoropropylene (FEP), polyesteramide (PEA), ethylene-tetrafiuoroethylene (ETFE), polyvinylidenefluoride (PVDF), polyether ether ketone (PEEK), low-density polyethylene (LDPE), and high-density polyethylene (HDPE).

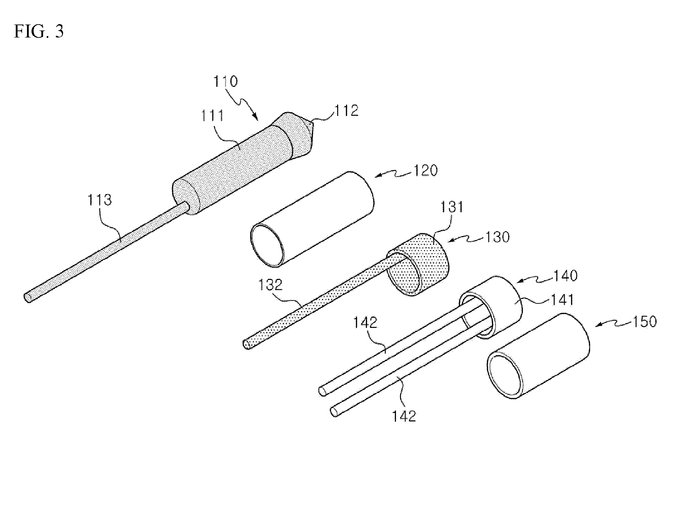

FIG. 2 is a perspective view showing the flexible body of the direction-controllable electrode body according to an embodiment of the present invention while FIG. 3 shows constituents of the flexible body of the direction-controllable electrode body according to an embodiment of the present invention.

With reference to FIGS. 2 and 3, the flexible body 101 comprises a first electrode 110, an insulator 120, a second electrode 130 and a direction controller, and optionally a first flexible protecting tube 150.

The first electrode 110 comprises a body 111, a first cap provided for one end of the body 111, and a first electrode line connected to the other end of the body 111. The body 111 serves as a support for the first electrode 110. So long as the body 111 supports the first electrode 110, no particular limitations are imparted to the morphology of the body 111. Among the constituents of the electrode 100, the first cap 112 is first inserted into the body. Although no particular limitations are imparted to the morphology of the first cap 112, it preferably has a wedge shape or semi-sphere shape. The first cap 112 may be further equipped with a minute thermometer for measuring the temperature of the position at which the electrode is located during the treatment. The first electrode line 113 is a steel wire which is preferably long enough to run through the flexible body 101. In addition, when a rigid body 102 is extended from the flexible body 101, the first electrode line 113 may also be extended to the end of the rigid body 102.

The first electrode 110 may be an anode or a cathode which is opposite to the polarity of the second electrode 130. So long as it allows a radiofrequency electric current to flow therethrough, any material may be used to form the first electrode 110. Preferably, the first electrode 110 may be made of metal, and more preferably one selected from the group consisting of stainless steel, alloy steel, titanium steel and shape memory alloy.

The insulator 120, which is in partial contact with the body 111 and the first cap 112 of the first electrode 110, functions to electrically insulate the first electrode 110 from the second electrode 130, that is, to prevent a short circuit between the first electrode 110 and the second electrode 130. Also, the insulator 120 fixes the first cap 112 of the first electrode 110 into the electrode body 100. Although drawn to be partially inserted into the body 111 and the first cap 112 in the figures, the insulator 120 may be connected in various other manners. So long as it is electrically non-conductive and thermally resistant, any material may be used to make the insulator 120. Preferably, the insulator 120 is made of a material selected from the group consisting of ceramic, silicon, a fluororesin, a heat-shrinkable polymer, and combinations thereof The ceramic may be exemplified by Al2O3. Representative among the silicon useful in the present invention is SiO2. Examples of the heat-shrinkable polymer include polytetrafluoroethylene (PTFE), tetrafluoroethylene-hexafluoropropylene (FEP), tetrafluoroethylene-perfluoroalkylvinylether (PFA), ethylene-tetrafluoroethylene (ETFE), polyester (PET), polyesteramide (PEA), and polyether ether ketone (PEEK).

The second electrode 130, which is in contact with the insulator 120, comprises a first ring 131 and a second electrode line 132 connected to one end of the first ring 131. Although the first ring 131, which is in direct contact with the insulator 120, is depicted as being inserted into the insulator 120 in the figure, the connection therebetween may be accomplished in various other manners. The first ring 131 is an output part, supplying electric power through the second electrode line 131. The second electrode line 132 is in the form of a steel wire which is long enough to run through the flexible body 11. In addition, when a rigid body 102 is extended from the flexible body 101, the second electrode line 131 may also be extended to the end of the rigid body 102.

The second electrode 130 may be an anode or a cathode which is opposite to the polarity of the first electrode 110. So long as it allows a radiofrequency electric current to flow therethrough, any material may be used to form the second electrode 130. Preferably, the second electrode 130 may be made of metal, and more preferably a material selected from the group consisting of stainless steel, alloy steel, titanium steel and shape memory alloy.

The direction controller 140, communicating with the first electrode 110 or the second electrode 130, comprises a first direction controlling wire 142. Also, the direction controller 140 may further comprise a second ring 141 which serves as an adaptor for connecting the direction controller 140 to the first electrode 110 or the second electrode 130. The second ring 141 may be connected to the first cap 111 of the first electrode 110 or the first ring 131 of the second electrode 130.

Herein, the first direction controlling wire 142 is preferably comprised of two or more wires. So long as it allows the flexible body 101 to be easily bent and has an electric resistance of from 0.1 µΩ to 5 Ω and a tensile strength of from 100 MPa to 20 GPa, any material may be used to make the first direction controlling wire. Examples of the material meeting these conditions include stainless steel, titanium, cobalt-chrome alloy, platinum, and silver. If it satisfies both the resistance and the tensile strength, the direction controlling wire 142 may be used to supply power in addition to providing direction control. In this case, the diameter of the electrode can be further reduced. Optionally, the first direction controlling wire 142 may be coated with enamel, that is, an insulating material. Examples of the insulating material include polytetrafluoroethylene (PTFE), tetrafluoroethylene-hexafluoropropylene (FEP), tetrafluoroethylene-perfluoroalkylvinylether (PFA), ethylene-tetrafluoroethylene (ETFE), polyester (PET), polyesteramide (PEA), and polyether ether ketone (PEEK).

The first direction controlling wire 142 is located at a phase of 180° in the second ring 141. As will be explained in detail later, the first direction controlling wire 142 communicates with a trigger 210 of a direction operator 200 and participates in controlling the direction of the electrode 100 with the operation of the trigger 210.

The flexible body 101 may further comprise the first flexible protecting pipe 150 to protect the first electrode 110, the insulator 120, the second electrode 130 and the direction controller 140. The first flexible protecting pipe 150 helps the flexible body 101 to bend. Preferably, the first flexible protecting pipe 150 is located beneath the first ring 131. When the direction controller 140 comprises the second ring 141, the first flexible protecting pipe 150 is preferably assembled to surround the entire surface of the second ring 141. So long as the first flexible protecting pipe 150 is readily bent by an operation force, its shape and material are not limited to specific ones. Preferably, it has a coil or articular structure. Further, the first flexible protecting pipe 150 is preferably made of a soft polymer that has a shore hardness of from 40 to 75 shore D. Among the soft polymers with a shore hardness of 40 to 75 shore D are polyamide resins such as Pebax 4533, Pebax 5533 and Pebax 7233 (Atochem), and nylon-12. Examples of such a soft polymer also include ultra high-molecular weight polyethylene (UHMP), polytetrafluoroethylene (PTFE), tetrafluoroethylene-hexafluoropropylene (FEP), polyesteramide (PEA), ethylene-tetrafluoroethylene (ETFE), polyvinylidenefluoride (PVDF), polyether ether ketone (PEEK), low-density polyethylene (LDPE), and high-density polyethylene (HDPE).

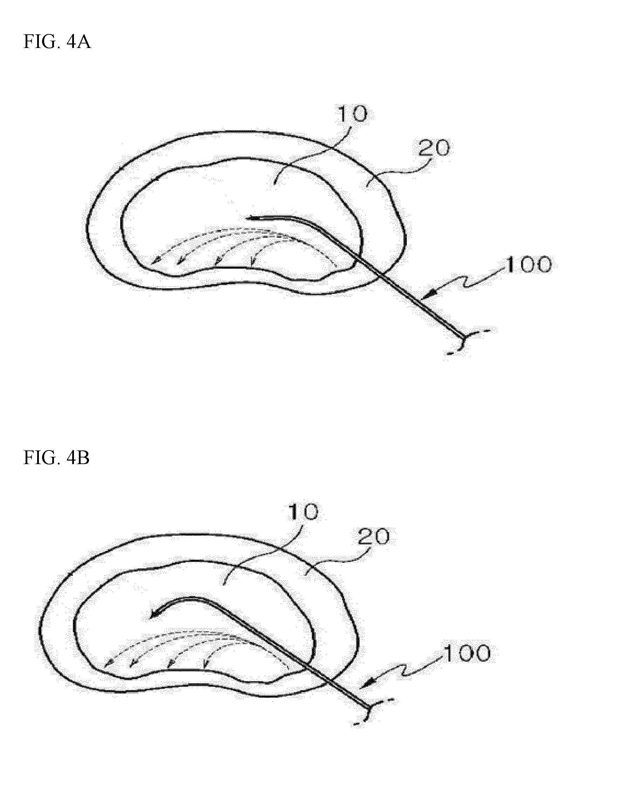

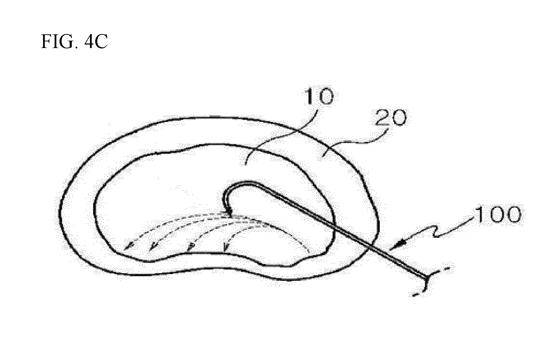

FIG. 4 shows a process of controlling the direction and position of the electrode body inserted into an intervertebral disc.

With reference to FIGS. 4A to 4C, the electrode body 100 is inserted into the nucleus pulposus 10 in the annulus fibrosus of an intervertebral disc while staying almost straight. After the insertion of the electrode body 100 into the body, the direction controller 140 is operated to bend the flexible body 101 of the electrode body 100 to move the cap 112, that is, the output portion of the electrode body 100, and the first ring 31 in a desired direction. As the direction controlling wire 142 of the direction controller 140 is pulled or allowed to become slack, the flexible body 101 is bent in a certain direction. In this manner, the cap 112 and the first ring 131 of the electrode body 100 can easily proceed toward their desired positions as shown by the arrows. Being connected to a radiofrequency generator, the direction controller 140 is also used to apply a radiofrequency to a target tissue through the output portion.

In accordance with an embodiment of the present invention, the electrode body may be connected with a direction operator at the rigid body's end opposite to the flexible body.

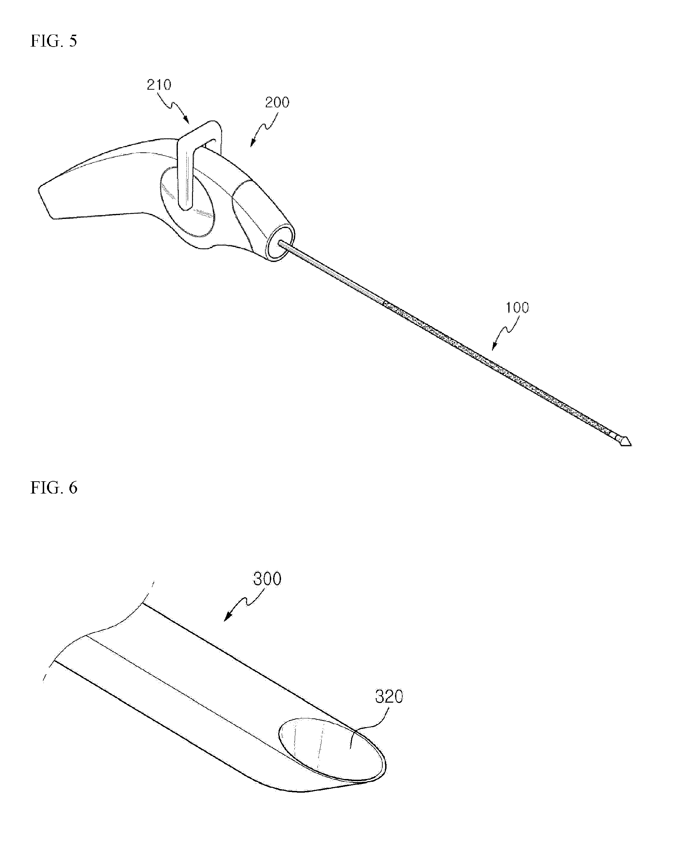

With reference to FIG. 5, an electrode body combined with a direction operator is shown in a perspective view.

As shown in FIG. 5, the direction operator 200 combined with the electrode 100 comprises a trigger 210. When one of the direction controlling wires 142 of the direction controller 140 is pulled by manipulating the trigger 210, the electrode body 100 is bent in the pulling direction. In this manner, the position and direction of the electrode body 100 can be controlled.

In accordance with an embodiment of the present invention, the electrode body may be contained in a curved needle having a groove at a lateral end. The curved needle helps the electrode body be more easily inserted into the body.

With reference to FIG. 6, a curved needle in which the electrode body is contained is shown in a perspective view.

As shown in FIG. 6, the curved needle 300 has a groove 320 in a lateral side and can control the motion of the electrode body more easily and accurately. In the absence of the curved needle 300, the electrode body must overcome the resistance of the internal substance of a disc in order to achieve a turnabout. On the other hand, when present in the curved needle, the electrode body can bend in a lateral direction at one end of the curved needle. Thus, the curved needle can significantly reduce the resistance of the internal substance of the disc to aid the turnabout of the electrode body. The curved needle may be made of stainless steel. The curved needle preferably has a conical form so that it does not tear the annulus fiber upon invasion.

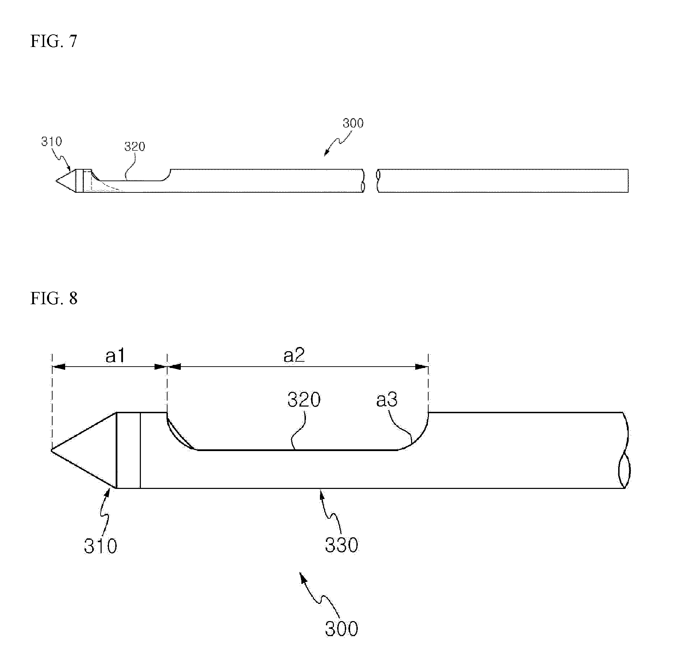

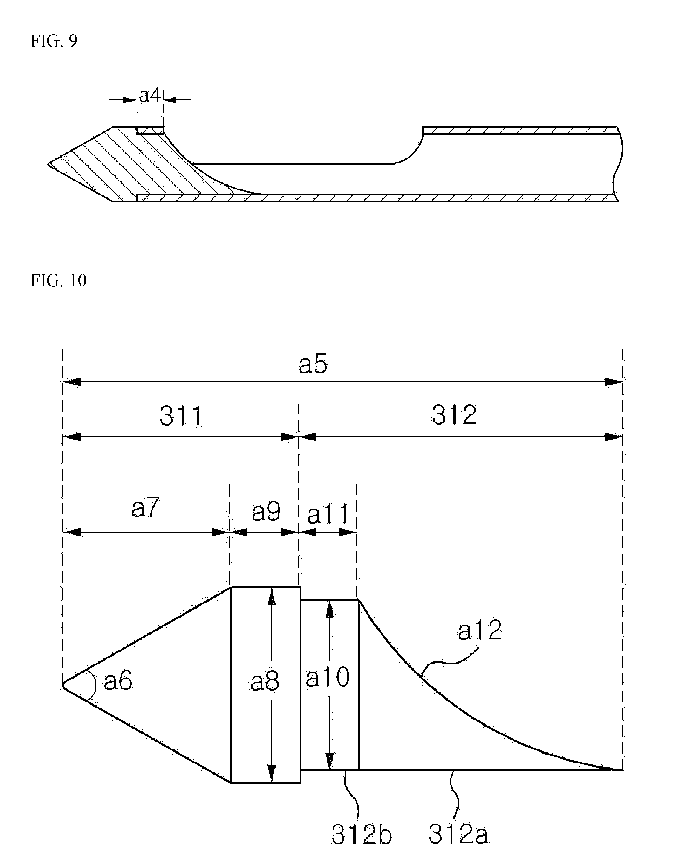

FIG. 7 is a side view of the curved needle having a conical cap at one end. FIG. 8 is a side view showing a part of the curved needle of FIG. 7 while FIG. 9 is a cross-sectional view showing a part of the curved needle of FIG. 7. FIG. 10 is a cross-sectional view of the conical cap of the curved needle.

As shown in FIG. 7 to 10, the curved needle 300 has a conical cap 310 at one end. The conical cap 310 is preferably connected to the main body by laser welding. In addition, the curved needle 300 has a groove 320 in one side so that it can readily bend in one direction. Preferably, the groove 320 has a round margin. In a preferred embodiment, the curved needle 300 is 190 mm - 210 mm in total length with an outer diameter of 1.50 mm - 2.00 mm and an inner diameter of 1.30 mm ∼ 1.40 mm. The length a1 from the conical cap 310 to the beginning of the groove 320 is preferably 2.00 mm to 3.20 mm and most preferably 2.50 mm. The groove length a2 preferably ranges from 5.50 mm to 5.80 mm. The groove 320 is rounded at margins with a preferable radius of 0.75 mm ∼ 0.82 mm and a most preferable radius of 0.80 mm. The length a4 between the conical cap 310 and the groove 320 is preferably 0.50 mm to 0.80 mm.

The conical cap 310 is composed of a cap part 311 and a fit part 312. To be fitted to the main body, the fit part 312 is designed to have a smaller thickness than that of the cap part 311. The total length a5 of the conical cap 310 is preferably 4.50 mm - 5.00 mm and most preferably 4.80 mm. The cap 311 is designed to have a conical portion and a cylindrical portion. The conical portion has an inner angle a6 of from 58° to 62° and a length a7 of from 1.25 mm to 1.55 mm. On the other hand, the cylindrical portion has a height a8 of from 1.50 mm to 1.70 mm and a length a9 of from 0.50 mm to 0.80 mm.

The fit part 312 is designed to have a support portion 312a and a contact portion 312b to be tightly fitted to the main body. The contact portion has a height a10 of from 1.25 mm to 1.35 mm and a length a11 of from 0.45 mm to 0.85 mm. Preferably, the support portion 312a has a straight line at a side in contact with the main body and a curved line elsewhere. So long as it does not obstruct the turnabout of the curved needle, any length may be given to the support portion without limitations. Preferably, the curved portion a12 has a radius of from 2.50 mm to 3.20 mm.

In accordance with an embodiment of the present invention, the direction of the electrode body is controlled while the electrode body is contained in a curved needle which has a groove in one side and a direction controlling wire installed within the groove.

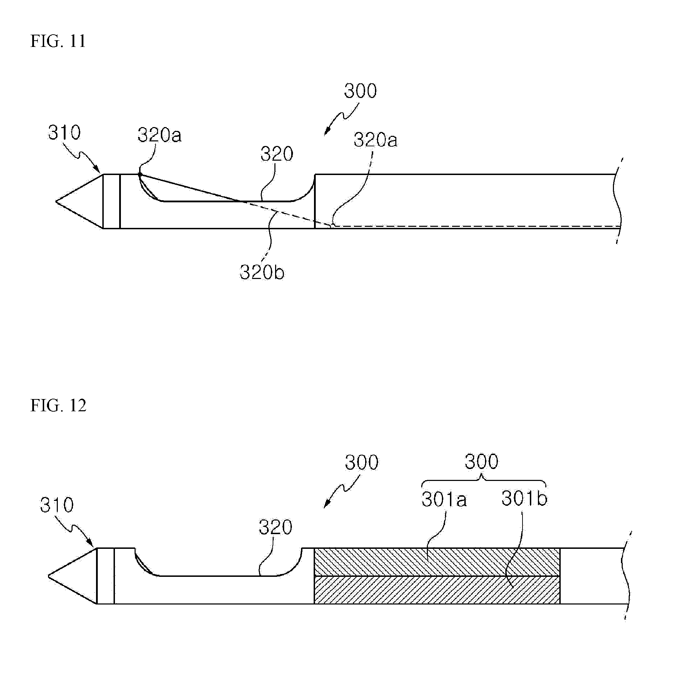

With reference to the side view of FIG. 11, a curved needle has a groove in one side and a wire installed within the groove, the wire being manipulated to control the direction of the curved needle.

In the main body of the curved needle, as shown in FIG. 11, an insertion hole 320a is formed at a position on an extension line of the straight line which links both ends of the groove 320. A wire 320a is inserted into the insertion hole 320a and extended in the direction opposite to the cylindrical cap 31to one end of the curved needle. By manipulating the wire 320b, the direction of the curved needle can be controlled.

In an embodiment of the present invention, the electrode body is inserted inside the curved needle which has a groove in one side and a flexible region adjacent to the groove so that the direction of the electrode body can be controlled. The flexible region of the curved needle makes it possible for the electrode body to reach a location which is difficult to access in terms of anatomical structure, particularly, due to the pelvic bone. The flexible region may be preferably made of one selected from the group consisting of a polymer, a metal or a composite of a polymer and a metal. The polymer may be fabricated into a pipe and examples of the polymer include ultra high-molecular weight polyethylene (UHMP), polytetrafluoroethylene (PTFE), tetrafluoroethylene-hexafluoropropylene (FEP), polyesteramide (PEA), ethylene-tetrafluoroethylene (ETFE), polyvinylidenefluoride (PVDF), polyether ether ketone (PEEK), low-density polyethylene (LDPE), and high-density polyethylene (HDPE).

The metal is preferably stainless steel and more preferably ASTM F899 type 304.

Further, the flexible region preferably has a structure selected from the group consisting of a cylindrical type, a meshed cylindrical type, a coil type and an articular type. Starting from the end of the conical cap 310, the length is preferably 0.2 mm∼1 cm to the beginning of the flexible region and 3cm∼10 cm to the end of the flexible region. If the conditions for the length are met, the curved needle can make an easy turnabout within the nucleus pulposus in the annulus fibrosus of a disc.

Referring to FIG. 12, there is a side view of a curved needle having a groove formed in one side and a cylindrical flexible region adjacent to the groove.

As shown in FIG. 12, the curved needle 300 has a cylindrical flexible region 301 which is formed at a position opposite to the cylindrical cap 310 and adjacent to the groove 320. The flexible region 301 is divided into a flexible polymer section 301a and a rigid metal section 301b. Preferably, the polymer section 301a is located in the same side that is for the groove 320 while the metal section 301b is located in a side opposite to that of the groove 320. The curved needle 300 having the cylindrical flexible region proceeds in virtue of the metal section 301b until the curved needle 300 penetrates into the annulus fibrosus while the polymer section 301a plays a role in turning the curved needle 300 in a different direction within the weakly resistant nucleus pulposus after penetration into the annulus fibrosus.

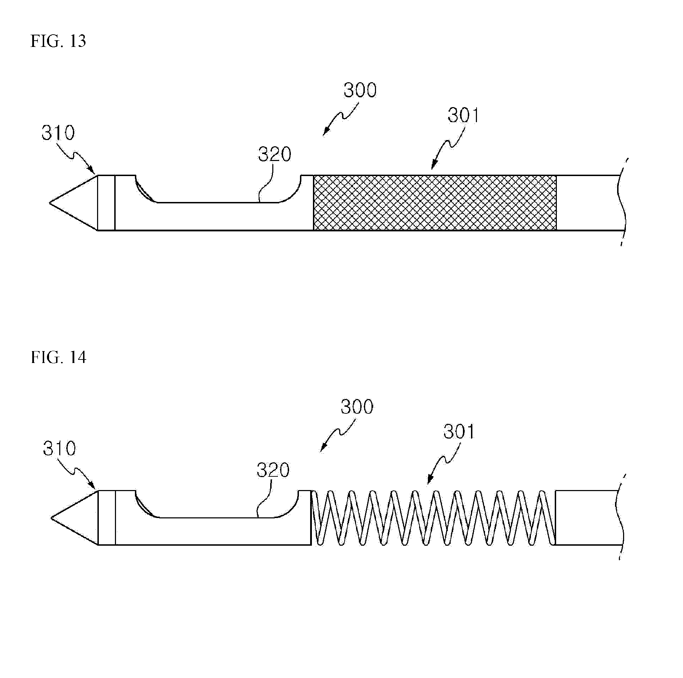

With reference to the side view of FIG. 13, a curved needle has a groove in one side and a mesh-type flexible region adjacent to the groove.

As shown in FIG. 13, the flexible region 301 is formed into a mesh structure in which an inner mesh-type metal structure is surrounded by a polymer. The mesh-type metal structure may be fabricated by laser cutting.

FIG. 14 is a side view of a curved needle having a groove in one side and a coil-type flexible region adjacent to the groove.

As shown in FIG. 14, the flexible region 301 is formed into a coil structure in which a coil-type metal structure is surrounded by a polymer.

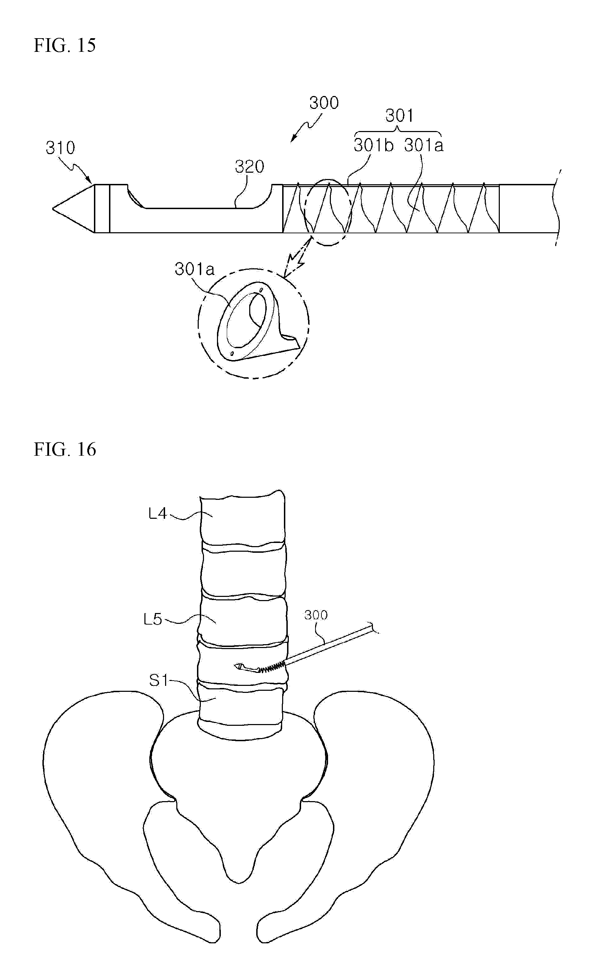

FIG. 15 is a side view of a curved needle having a groove in one side and an articular flexible region adjacent to the groove.

As shown in FIG. 15, the flexible region has an articular type structure which is preferably composed of a plurality of triangular rings. In each triangular ring, an insertion hole or a recess into which a direction controlling wire is inserted is preferably formed at an upper and a lower position.

FIG. 16 is a schematic view showing a curved needle having a groove in one side and a flexible region adjacent to the groove which is inserted into the disc between the fifth lumbar vertebra L5 and the sacrum S 1.

As seen in FIG. 16, the curved needle 300 is inserted into a disc in an oblique direction and the flexible region 301 is bent into the center of the disc within the nucleus pulposus.

In accordance with an embodiment of the present invention, the electrode body is associated with a protector for protecting a body tissue around the electrode body from the heat and current generated from the electrode body. The protector may be contained together with the electrode body within the curved needle having a groove in one side. Intercalated between the disc and the nerve, the protector acts to protect bodily tissue around the electrode body from the heat and electric current generated by the electrode body.

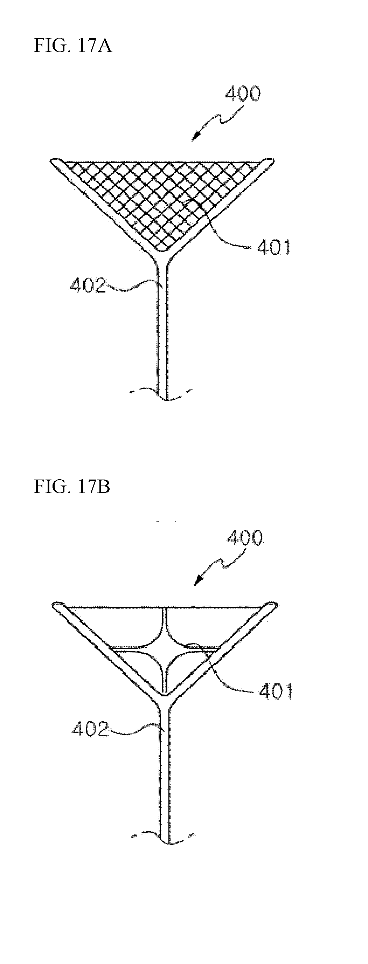

The side views of FIG. 17 show protectors for protecting bodily tissue from the heat generated by the electrode body.

As seen in FIG. 17, the protector 400 comprises a protecting membrane 401 and a support 402 for supporting the protecting membrane 401. The protecting membrane 401 may be preferably a mesh type (a) or a cruciform type (b). No specific limitations are imparted to the material of the protecting membrane 401 if it can be readily folded and spread. Preferably, the protecting membrane 401 is made of a material selected from the group consisting of polytetrafluoroethylene (PTFE), polyethylene (PE), polyether ether ketone (PEEK), tetrafluoroethylene-hexafluoropropylene (FEP), tetrafluoroethylene-perfluoroalkylvinylether (PFA), ethylene-tetrafluoroethylene (ETFE), polyimide (PI), polyester (PET), polyesteramide (PEA), polyamide(PA) and combinations thereof. The support 402 is preferably a flexible Y-shaped tube. The support 402 may be of an I-shaped structure within the curved needle and may be transformed into a Y-shaped structure when it is outside the curved needle. No particular limitations are imparted to the material of the support 402 so long as it is neither sensitive to heat nor toxic and does not burst into fragments. Preferably, the support may be made of a polymer, a metal or a composite of a polymer and a metal. More preferably, it is made of a polymer selected from the group consisting of polytetrafluoroethylene (PTFE), polyethylene (PE), polyether ether ketone (PEEK), tetrafluoroethylene-hexafluoropropylene (FEP), tetrafluoroethylene-perfluoroalkylvinylether (PFA), ethylene-tetrafluoroethylene (ETFE), polyimide (PI), polyester (PET), polyesteramide (PEA), polyamide (PA) and combinations thereof The metal may be preferably stainless steel.

When the same material is used for the protecting membrane 401 and the support 402, they may be preferably fabricated from a mold by injection molding without a special joining process. If different materials are used therefor, the protecting membrane 401 and the support 402 may be consolidated together by melting and pressing, or may be mechanically joined using a thread, a string or a tack. Alternatively, the support may be fabricated by intercalating the protecting membrane between two moieties of the support and joining the two moieties through an interference fit.

Below, a description is given of a method for treating disc disease using the electrode body.

The method for treating disc disease in accordance with the present invention comprises:

a step of (A) approaching a location of a lesion causing back pain by controlling the direction controllable electrode body, and conducting at least one step selected from the group consisting of (B) searching a nerve responsible for the pain by stimulating the lesion with the electrode body; (C) treating the disc disease by coagulating the lesion with the electrode body; and (D) treating the disc disease by ablating a disc tissue causing the pain with the electrode body.

In the method for treating disc disease, the steps may be performed in a variety of different orders. For example, step (A) may be followed by step (D).

Alternatively, the steps may be conducted in the order of (A), (B) and (C).

In another alternative, step (A) is conducted, followed by sequentially conducting steps (D), (B) and (C).

The steps of the method according to the present invention will be explained in greater detail, below.

Step (A) of approaching a location of a lesion causing back pain may be performed by inserting the electrode body into the annulus fibrosus of an intervertebral disc of interest, with the electrode body staying spread, and controllably bending the flexible body of the electrode body in a desired direction to position the cap and the ring on the lesion. To bend the flexible body in a desired direction, an operator conjugated with the electrode body is used. As the trigger of the operator is pulled or unfastened, the flexible body may be bent in a certain direction. In this manner of operation, the output portion of the electrode, that is, the cap and the ring may be readily positioned on the lesion.

Step (B) of searching a nerve responsible for the pain may comprise applying an alternating current of 1 Hz to 300 Hz at a voltage of from 0.1 to 3.0 V to the electrode body. In this way, a nerve responsible for the pain can be readily detected.

Step (C) of treating the disc disease by coagulating the lesion with the electrode body in which an alternating current of 300 - 500 kHz is applied to the electrode body to remove the nerve.

Step (D) of ablating a disc tissue may be conducted by applying an alternating current of 300 kHz - 1 MHz to the electrode body at a voltage of 50 ∼ 800 V to generate plasma.

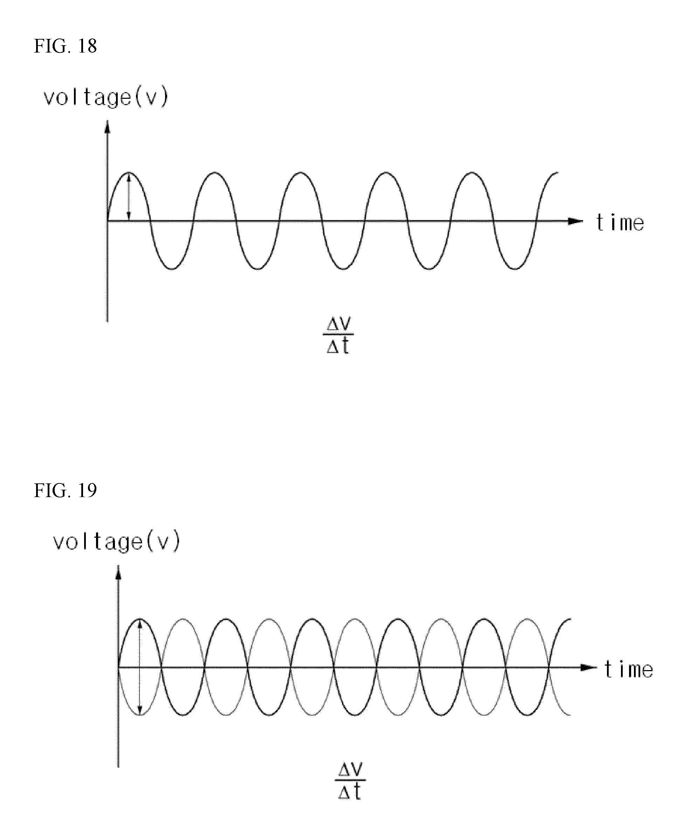

In order to generate plasma with maximal efficiency in step (D), it is necessary to rapidly increase the voltage of the alternating current within a short time. In this context, the use of two waveforms with a phase difference of 180° can increase the voltage at a rate twice as high as can the use of one waveform. This effect is shown in the graphs of FIGS. 18 and 19.

FIG. 18 shows a waveform generated when one alternating current is used while FIG. 19 shows waveforms generated when two alternating currents are used with a phase difference. Herein, the Y-axis represents a voltage (unit: V) and the X axis represents time.

As can be seen in FIGS. 18 and 19, a voltage upon the use of two waveforms is twice as high as that upon the use of one waveform.

Particularly, when alternating currents with a phase difference of 180° are applied at 300 ∼ 500 kHz to the electrode body of the present invention, the efficiency of plasma generation can be further increased. Also, the efficiency of plasma generation can be increased by generating two electric currents at the same time in a different phase irrespective of the waveforms of the electric currents applied.

The treatment method using the electrode body in accordance with the present invention allows the local ablation of even a herniated intervertebral disc which is difficult to approach in a straight manner. For example, after the removal of the nucleus pulposus from an intervertebral disc by use of the electrode body of the present invention, a balloon is inserted inside the disc through a guide pipe and a suitable pressure is applied to the balloon to stabilize the balloon within the disc. In this case, an artificial nucleus pulposus may be injected within the balloon.

In addition to disc disease, the electrode body of the present invention may be applied to the place from which bodily tissue is locally removed. For example, it may be used to remove a tumor, cancer, thrombi, intravascular plaques, vessel stenosis, fibroma, uterine myoma, a sweat gland for treating osmidrosis axillae, intestinal polyps, intragastric lumps, urethral stricture, cartilaginous stenosis, excessively grown nerve tissues, etc.

In accordance with another aspect thereof, the present invention provides a direction-controllable guide pipe. For use in suction or irrigation, the guide pipe may be inserted into the body. Further, when equipped with a lens and other suitable devices, the guide pipe may be used as an endoscope. Also, the guide pipe may be applied to the removal of a herniated intervertebral disc when associated with the electrode body of the present invention. In this case, the guide pipe can lead the electrode body to a desired position thanks to its excellent rigidity and excellent ability to have its direction controlled.

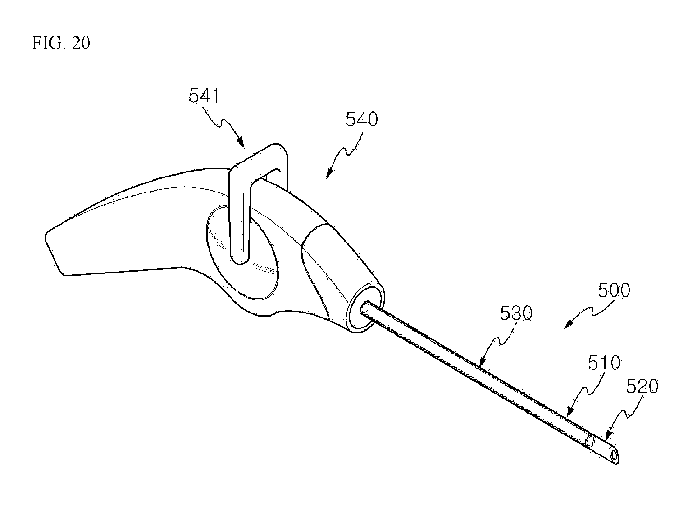

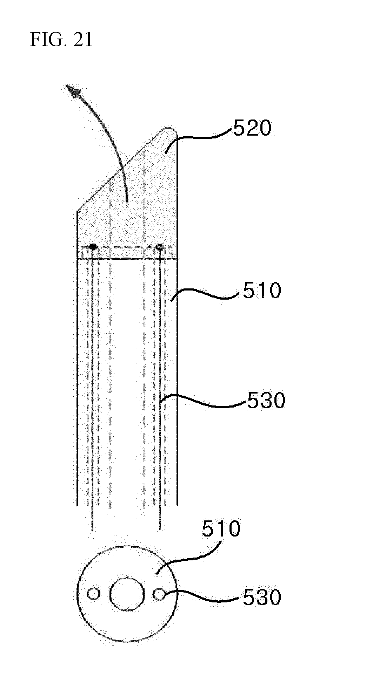

FIG. 20 is a perspective view of a guide pipe according to an embodiment of the present invention and FIG. 21 is an enlarged cross-sectional view showing the structure of the guide pipe of FIG. 20.

As shown in FIGS. 20 and 21, the guide pipe 500 comprises a second flexible protecting pipe 510, a second cap 520, a second direction controlling wire 530 and a second direction operator.

The second flexible protecting pipe 510 preferably has a coil or articular structure. A description of the coil or articular structure is as given to the flexible region of the curved needle. The second flexible protecting pipe 510 may be preferably made of a soft polymer which ranges in shore hardness from 40 to 75 shore D. The soft polymer with a shore hardness of 40 ∼ 75 shore D is preferably selected from the group consisting of Pebax 4533, Pebax 5533, Pebax 7233(Atochem), Nylon-12, ultra high-molecular weight polyethylene (UHMP), polytetrafluoroethylene (PTFE), tetrafluoroethylene-hexafluoropropylene (FEP), polyesteramide (PEA), ethylene-tetrafluoroethylene (ETFE), polyvinylidenefluoride (PVDF), polyether ether ketone (PEEK), low-density polyethylene (LDPE), high-density polyethylene (HDPE) and combinations thereof.

So long as the second cap 520 conjugated to one end of the second flexible protecting pipe 510 can be inserted into the body, no particular limitations are imparted to the morphology of the cap 520. Preferably, it has a wedge shape or semi-sphere shape which can reduce the resistance upon insertion. The second cap 520 may be preferably made of one selected from the group consisting of stainless steel, alloy steel, titanium steel and shape memory alloy, and more preferably made of stainless steel.

The second direction controlling wire 530 starts from the second cap 520, and runs through the second flexible protecting pipe 510 to the other end of the protecting pipe 510. The second direction controlling wire 530 can be used in direction control in the same manner as in the first direction controlling wire of the electrode body according to the present invention. Further, the second direction controlling wire is preferably composed of at least two wires. Optionally, the second direction controlling wire 530 may be coated with enamel, that is, an insulating material. Examples of the insulating material include polytetrafluoroethylene (PTFE), tetrafluoroethylene-hexafluoropropylene (FEP), tetrafluoroethylene-perfluoroalkylvinylether (PFA), ethylene-tetrafluoroethylene (ETFE), polyester (PET), polyesteramide (PEA), and polyether ether ketone (PEEK).

Communicating with the second direction controlling wire 530, the second direction operator 540 is manipulated to pull the second direction controlling wire 530 to control the direction of the guide pipe. The second direction operator 540 is of the same description as was given for the first direction operator 200.

The guide pipe 500 may have an inner diameter of 0.5 ∼ 2.5 mm and an outer diameter of 1 ∼ 3 mm. An outer diameter greater than 3 mm may cause another significant trauma in the annulus fibrosus of the disc upon insertion.

In an embodiment of the present invention, the guide pipe may have a trocar therein.

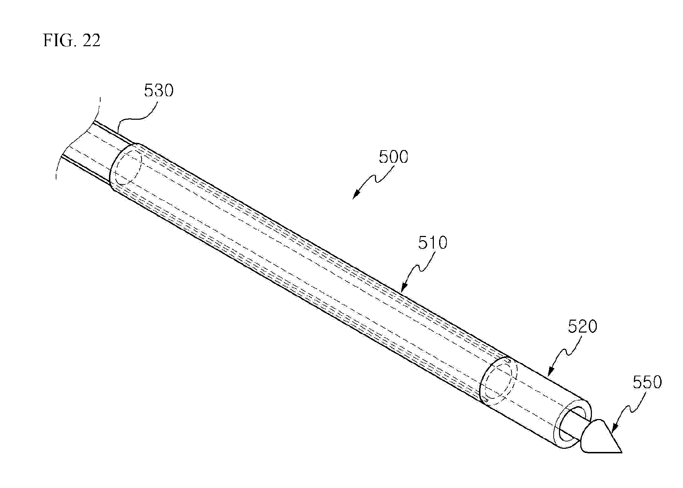

FIG. 22 is a perspective view showing a guide pipe into which a trocar is inserted.

As shown in FIG. 22, a trocar 550 is contained within the guide pipe 500. So long as the trocar 550 is anyone that is used in the medical field, no particular limitations are imparted to the shape and material of the trocar. When the trocar 550 is inserted thereinto, the guide pipe stays straight. When the trocar is removed therefrom, the guide pipe may exhibit flexibility. The trocar 550 is located within the second flexible protecting pipe to prevent bodily materials from clogging the guide pipe when the guide pipe invades the body. After the insertion of the guide pipe 500 containing the trocar 550 into the body, the trocar is removed and the guide pipe is manipulated in a direction-controlled manner to advance to the target point. The employment of the trocar 550 makes it possible for the electrode body to readily approach a lesion without the use of a needle such as a curved needle.

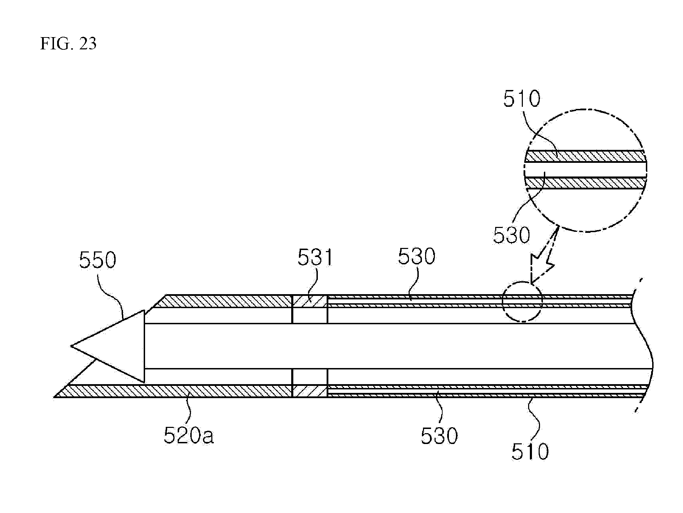

FIG. 23 is a cross-sectional view showing a guide pipe comprising a second direction controlling wire extended from a third ring, with a trocar inserted thereinto, in accordance with an embodiment of the present invention.

As seen in FIG. 23, a third ring 531 is intercalated between the second cap 520 and the second flexible protecting pipe 510 while the second direction controlling wire 530 extends from the third ring 531 and runs through the second flexible protecting pipe 510. Further, a trocar 550 is inserted inside the second flexible protecting pipe 510. Preferably, the second cap 520 is made of a metal while the second flexible protecting pipe 510 is made of a soft polymer.

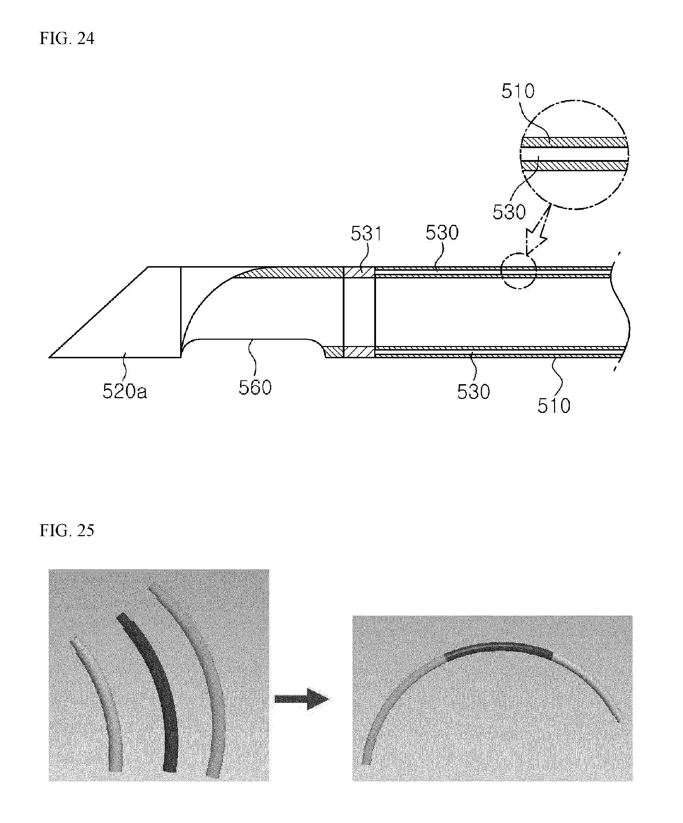

FIG. 24 is a cross-sectional view of a guide pipe in which a groove is formed between the second cap and the third ring, but not at one end of the second cap, in one side.

As seen in FIG. 24, a groove 560 is formed on one side of the guide pipe, so that the electrode according to one embodiment of the present invention can be released through the groove 560 and brought into contact with the body.

A plurality of the guide pipes of the present invention may be assembled into a hollow pipe with a radius of curvature and a length, as shown in FIG. 25. Before application, the guide pipes may be assembled, like hula hoop blocks, to construct a desired path which can effectively guide the electrode body to a target point. In this context, the guide pipe 500 may preferably have a radius of curvature of from 10 to 5000 mm, a length of from 5 to 500 mm, and a diameter of from 0.5 to 2.5 mm. The guide pipe 500 may be made of a rigid material, such as stainless steel, a flexible material such as a soft polymer, or combinations thereof.

In accordance with an embodiment of the present invention, the guide pipe may be directed in a controlled manner with the electrode body contained therein.

## Claims

1. A direction controllable electrode body, comprising a flexible body which comprises:
a first electrode, comprising a body, a first cap joined to one end of the body, and a first electrode line connected to the other end of the body;
an insulator partly contact with the body and the first cap of the first electrode, said insulator functioning to insulate the first electrode from the second electrode;
a second electrode, comprising a first ring in contact with the insulator, and a second electrode line connected to one end of the first ring; and
a direction controller, comprising a first direction controlling wire communicating with the first electrode or the second electrode to control direction of the electrode body.

2. The direction controllable electrode body of claim 1, further comprising a rigid body joined to the flexible body.

3. The direction controllable electrode body of claim 2, further comprising a first direction operator combined with the rigid body.

4. The direction controllable electrode body of claim 1, wherein the body of the first electrode is integrated with the first cap.

5. The direction controllable electrode body of claim 1, wherein the first electrode and the second electrode are independently made of a material selected from the group consisting of stainless steel, general alloy steel, titanium steel and shape memory steel.

6. The direction controllable electrode body of claim 1, wherein the insulator is made of a material selected from the group consisting of ceramic, silicon, a fluororesin, a heat-shrinkable polymer, and combinations thereof

7. The direction controllable electrode body of claim 6, wherein the ceramic is Al₂O₃, the silicon is SiO₂, and the heat-shrinkable polymer is selected from the group consisting of polytetrafluoroethylene (PTFE), tetrafluoroethylene-hexafluoropropylene (FEP), tetrafluoroethylene-perfluoroalkylvinylether (PFA), ethylene-tetrafluoroethylene (ETFE), polyester (PET), polyether ether ketone (PEEK), and combination thereof.

8. The direction controllable electrode body of claim 1, wherein the first direction controlling wire has an electric resistance of from 0.1 µΩ to 5 Ω and a tensile strength of from 100 MPa to 20 GPa and is used both to control direction of the electrode body and to serve as a power supply line, whereby a diameter of the electrode can be reduced.

9. The direction controllable electrode body of claim 8, wherein first direction controlling wire is made of a material selected from the group consisting of stainless ste el, titanium, cobalt-chrome alloy, platinum, silver and combinations thereof

10. The direction controllable electrode body of claim 1, wherein the first direction controlling wire is coated with enamel.

11. The direction controllable electrode body of claim 1, wherein the direction controller comprises a second ring connected to the first electrode or the second electrode.

12. The direction controllable electrode body of claim 11, wherein the second ring is connected to the first cap of the first electrode or the first ring of the second electrode.

13. The direction controllable electrode body of claim 1, wherein the flexible body further comprises a first flexible protecting pipe for protecting the first electrode, the insulator, the second electrode and the direction controller.

14. The direction controllable electrode body of claim 13, wherein the first flexible protecting pipe has a coil or articular structure.

15. The direction controllable electrode body of claim 13, wherein the first flexible protecting pipe is made of a soft polymer.

16. The direction controllable electrode body of claim 15, wherein the soft polymer has a shore hardness of from 40 to 75 shore D.

17. The direction controllable electrode body of claim 16, wherein the soft polymer is selected from the group Pebax 4533, Pebax 5533, Pebax 7233 (Atochem), Nylon-12, ultra high-molecular weight polyethylene (UHMP), polytetrafluoroethylene (PTFE), tetrafluoroethylene-hexafluoropropylene (FEP), polyesteramide (PEA), ethylene-tetrafluoroethylene (ETFE), polyvinylidenefluoride (PVDF), polyether ether ketone (PEEK), low-density polyethylene (LDPE), high-density polyethylene (HDPE) and combinations thereof

18. The direction controllable electrode body of claim 1, being inserted into a groove formed in one side of a curved needle, whereby the electrode body can be directionally controlled as the curved needle is operated.

19. The direction controllable electrode body of claim 18, wherein the curved needle has a flexible region adjacent to the groove.

20. The direction controllable electrode body of claim 19, wherein the flexible region is made of a material selected from the group consisting of a polymer, a metal and a composite of polymer and metal.

21. The direction controllable electrode body of claim 20, wherein the polymer is selected from the group consisting of ultra high-molecular weight polyethylene (UHMP), polytetrafluoroethylene (PTFE), tetrafluoroethylene-hexafluoropropylene (FEP), polyesteramide (PEA), ethylene-tetrafluoroethylene (ETFE), polyvinylidenefluoride (PVDF), polyether ether ketone (PEEK), low-density polyethylene (LDPE), high-density polyethylene (HDPE) and combinations thereof, and the metal is stainless steel.

22. The direction controllable electrode body of claim 20, wherein the flexible region has a structure selected from the group consisting of cylindrical type, a meshed cylindrical type, a coil type and an articular type.

23. The direction controllable electrode body of claim 22, wherein the cylindrical type structure of the flexible region has one side made of a metal and the other side made of a polymer and the curved needle is bent in the direction of the polymer side.

24. The direction controllable electrode body of claim 22, wherein the articular type structure of the flexible region is composed of a plurality of triangular rings.

25. The direction controllable electrode body of claim 24, where each of the triangular rings has an insertion hole or a recess formed at an upper and a lower position thereof into which a direction controlling wire is inserted.

26. The direction controllable electrode body of claim 1, being associated with a protector for protecting an adjacent body tissue from heat generated from the electrode body, said protector comprising a protecting membrane and a support for supporting the protecting membrane.

27. The direction controllable electrode body of claim 26, wherein the protecting membrane has a mesh type structure or a cruciform type structure.

28. The direction controllable electrode body of claim 26, wherein the protecting membrane is made of a material selected from the group consisting of polytetrafluoroethylene (PTFE), polyethylene (PE), polyether ether ketone (PEEK), tetrafluoroethylene-hexafluoropropylene (FEP), tetrafluoroethylene-perfluoroalkylvinylether (PFA), ethylene-tetrafluoroethylene (ETFE), polyimide (PI), polyester (PET), polyamide (PA) and combinations thereof

29. The direction controllable electrode body of claim 26, wherein the support is a Y-shaped tube.

30. The direction controllable electrode body of claim 26, wherein the support is made of a material selected from the group consisting of a polymer, a metal and a composite thereof

31. The direction controllable electrode body of claim 30, wherein the polymer is selected from the group consisting of polytetrafluoroethylene (PTFE), polyethylene (PE), polyether ether ketone (PEEK), tetrafluoroethylene-hexafluoropropylene (FEP), tetrafluoroethylene-perfluoroalkylvinylether (PFA), ethylene-tetrafluoroethylene (ETFE), polyimide (PI), polyester (PET), polyamide (PA), and combinations thereof

32. The direction controllable electrode body of claim 30, wherein the metal is stainless steel.

33. A method for treating disc disease, comprising:
a step of (A) approaching a location of a lesion causing back pain by controlling the direction controllable electrode body of claim 1; and
at least one step selected from the group consisting of (B) searching a nerve responsible for the pain by stimulating the lesion with the electrode body; (C) treating the disc disease by coagulating the lesion with the electrode body; and (D) treating the disc disease by ablating a disc tissue causing the pain with the electrode body.

34. The method of claim 33, wherein the step (A) is followed by step (D).

35. The method of claim 33, wherein the steps (A), (B) and (C) are conducted in that order.

36. The method of claim 33, wherein the step (A) is conducted, followed by sequentially conducting steps (D), (B) and (C) in that order.

37. The method of claim 33, wherein the step (A) of approaching a location of a lesion causing back pain comprises:
inserting the electrode body into the annulus fibrosus of an intervertebral disc of interest, with the direction controllable electrode body staying spread; and
controllably bending the flexible body of the electrode body in a desired direction to position the cap and the ring on the lesion.

38. The method of claim 33, wherein the step (B) of searching a nerve responsible for the pain by stimulating the lesion with the electrode body comprises applying an alternating current of 1 Hz to 300 Hz at a voltage of from 0.1 to 3.0 V to the electrode body to detect a nerve responsible for the pain.

39. The method of claim 33, wherein the step (C) of coagulating the lesion is conducted by electrocautery in which an alternating current of 300 - 500 kHz is applied to the electrode body to remove the nerve.

40. A direction controllable guide pipe, comprising:
a second flexible protecting pipe;
a second cap conjugated to one end of the second flexible protecting pipe;
a second direction controlling wire, extending from the second cap to the other end of the second flexible protecting pipe; and
a hollow, second direction operator communicating with the second direction controlling wire.

41. The guide pipe of claim 40, wherein the second flexible protecting pipe has a coil type or articular type structure.

42. The guide pipe of claim 40, wherein the second flexible protecting pipe is made of a soft polymer.

43. The guide pipe of claim 42, wherein the soft polymer has a shore hardness of 40 ∼ 75 shore D.

44. The guide pipe of claim 43, wherein the soft polymer is selected from the group consisting ofPebax 4533, Pebax 5533, Pebax 7233 (Atochem), Nylon-12, ultra high-molecular weight polyethylene (UHMP), polytetrafluoroethylene (PTFE), tetraffuoroethylene-hexafluoropropylene (FEP), polyesteramide (PEA), ethylene-tetrafluoroethylene (ETFE), polyvinylidenefluoride (PVDF), polyether ether ketone (PEEK), low-density polyethylene (LDPE), high-density polyethylene (HDPE), and combinations thereof

45. The guide pipe of claim 40, wherein the second direction controlling wire has an electric resistance of from 0.1 µΩ to 5 Ω and a tensile strength of from 100 MPa to 20 GPa and is used both to control direction of the direction controllable electrode body and to serve as a power supply line, whereby a diameter of the electrode can be reduced.

46. The guide pipe of claim 45, wherein the second direction controlling wire is made of a material selected from the group consisting of stainless steel, titanium, cobalt-chrome alloy, platinum, silver and combinations thereof

47. The guide pipe of claim 40, wherein the second direction controlling wire is coated with enamel.

48. The guide pipe of claim 40, containing a trocar therein.

49. The guide pipe of claim 40, containing the direction controllable electrode body of claim 1 therein, whereby the direction controllable electrode body can be allowed to make a turnaround in a controlled manner established by the guide pipe.

50. The guide pipe of claim 40, which has a radius of curvature and a length and serves as a segment many of which are assembled into a larger guide pipe.

51. The guide pipe of claim 50, which has a radius of curvature ranges from 10 to 5000 mm, a length ranges from 5 to 500 mm and a diameter ranges from 0.5 to 3.5 mm.

52. The guide pipe of claim 40, containing the direction controllable electrode body of claim 1 therein.
